# FASCICULE DE BREVET EUROPEEN

(11) **EP 0 652 963 B1**
(45) Date de publication et mention de la délivrance du brevet: **24.09.1997**
(21) Numéro de dépôt: 93915577.6
(22) Date de dépôt: 02.08.1993
(51) Int. Cl.: C12N 15/64, C12N 1/21

(54) **VECTEUR DE CLONAGE ET/OU DE SEQUENCAGE**
VEKTOR FÜR DIE KLONIERUNG UND/ODER SEQUENZIERUNG
CLONING AND/OR SEQUENCING VECTOR

(30) Priorité: 31.07.1992 BE 9200696
(43) Date de publication de la demande: 17.05.1995
(73) Titulaire: UNIVERSITE LIBRE DE BRUXELLES, B-1050 Bruxelles (BE)
(72) Inventeur: BERNARD, Philippe, B-1060 Bruxelles (BE); GABANT, Philippe, B-1030 Bruxelles (BE)
(74) Mandataire: Van Malderen, Joelle
(86) Numéro de dépôt international: BE9300051
(87) Numéro de publication internationale: WO9403616

(56) Documents cités:
- MOLECULAR & GENERAL GENETICS, vol. 226, no. 1/2, April 1991, Springer International, New York, NY (US); P. BERNARD et al., pp. 297-304
- JOURNAL OF CELLULAR BIOCHEMISTRY, suppl. 0, vol. 16b, 1992; M. COUTURIER et al., p. 104
- PROCEEDINGS OF THE NATL. ACADEMY OF SCIENCES, vol. 89, no. 6, 15 March 1992, Washington, DC (US); J.C. PIERCE et al., pp. 2056-2060
- GENE, vol. 42, no. 3, 1986, Elsevier Publishers, New York, NY (US); B. HENRICH et al., pp. 345-349; and I. KUHN et al., pp. 253-263
- MOLECULAR CLONING, LABORATORY MANUAL, 2nd ed., vol. 1-3, 1989, CSH, Long Island, NY (US); SAMBROOK et al., pp. 4.12-A9/A13
- JOURNAL OF MOLECULAR BIOLOGY, vol. 226, no. 3, 05 August 1992, Academic Press Limited, London (GB); P. BERNARD et al., pp. 735-745

## Description

### Objet de l'invention

L'invention concerne un vecteur de clonage et/ou de séquençage permettant une sélection directe de clones recombinants.

L'invention concerne également la cellule procaryote transformée par ce vecteur et la cellule procaryote hôte de ce vecteur ainsi que l'utilisation de ce vecteur de clonage et de séquençage pour la sélection et le séquençage de clones recombinants.

### Etat de la technique et arrière plan technologique à la base de l'invention

Des vecteurs de clonage phagiques (série des M13) et plasmidiques (série des pUC) et contenant de nombreux sites uniques de clonage ont été construits par Messing et al (P.N.A.S. USA, 79, p 3642-3646 (1977), par Norrander et al (Gene, 26, p 101-106 (1983) et Yanisch-perron et al (Gene, 33 p 103 à 119 (1985)).

Les multiples sites de clonage (MCS - multiples cloning sites) de ces vecteurs sont localisés dans la séquence codante du gène *Lac*Z*.*

La discrimination entre les cellules transformées hébergeant un vecteur recombinant et les cellules hébergeant un vecteur non recombinant s'effectue par la technique du "Blue Screen" décrite par Gronenborn et Messing (Methylation of single-stranded DNA in vitro introduces new restriction endonuclease cleavages sites, Nature, 272, p 375-377 (1978)).

Cependant, cette technique du "Blue Screen" présente l'inconvénient d'utiliser un procédé de criblage (descrimination) et non un procédé de sélection des clones.

La discrimination par criblage est basée sur l'identification d'un clone au sein d'une population de clones sur base d'une caractéristique (couleur) qui le différencie. La sélection ne nécessite pas cette caractéristique, puisque par cette méthode, on isole uniquement les clones recombinants.

Le procédé de criblage est basé sur la coloration des clones recombinants (coloration blanche) et des clones non recombinants (coloration bleue). Cette coloration est basée sur l'inactivation du marqueur Beta-galactosidase, prévenant le clivage de l'X-gal (5-bromo-4-chloro-3-indolyl-b-galactoside). Les colonies cellulaires hébergeant un vecteur non recombinant produisent une Beta-galactosidase fonctionnelle et en hydrolysant le substrat X-gal, produisent un colorant bleu. L'insertion d'un fragment d'ADN dans le gène de la β-galactosidase, prévient généralement le clivage de l'X-gal. Les cellules hébergeant un vecteur recombinant ont donc une coloration blanche.

En outre, ce procédé complexe nécessite l'utilisation du substrat X-gal qui est un produit fort coûteux, instable et d'utilisation délicate.

D'autre part, divers vecteurs de clonage permettant de sélectionner directement (sélection positive) des souches recombinantes ont été décrits dans la littérature scientifique.

Pierce et al (Proc. Natl. Acad. Sci., vol 89, n°6 1992, p 2056-2060) décrivent un vecteur comprenant le gène letal sacB de Bacillus amyloliquefaciens, intégré dans un plasmide dérivé du bactériophage P1, sous le contrôle d'un promoteur spécifique de E. coli.

Le promoteur de ce vecteur comporte une région avec plusieurs sites spécifiques de clonage (site de clivage pour une enzyme de restriction).

Comme le gène sacB code pour le levansucrase catalysant l'hydrolyse du sucrose en produits toxiques pour E. coli; la sélection directe des mutants intégrant un plasmide recombiné, se fait sur une milieu de culture contenant du sucrose. Comme le levansucrase est toxique, même en l'absence de sucrose, il est donc indispensable de réprimer sa synthèse si l'on désire obtenir un grand nombre de copies plasmidiques dans le cytoplasme bactérien.

Cependant une repression totale du gène cytotoxique est difficile, voire impossible, particulièrement si on désire avoir un grand nombre de copies du vecteur.

Aussi, l'impossibilité de réprimer le gène cytotoxique entraîne dans les phases de production du plasmide, la mort de la cellule et par conséquent une pression sélective vers des souches mutées (caractérisées par un gène letal inactif).

Dans ce cas ci, pour que l'enzyme codée par le gène sac B ne tue pas la cellule hôte, il est nécessaire d'incorporer au vecteur de clonage un répresseur CI qui régule l'expression de ce gène.

De plus, comme le sucrose est souvent incorporé au milieux de culture bactériens, il sera indispensable de préparer des milieux totalement exempts de sucrose pour pratiquer ces manipulations.

Henrich et al (Gene, vol 42, n°3, 1986, p 345-349) décrivent un vecteur comprenant le gène E issu du bactériophage φX174, ledit gène E étant incorporé dans le plasmide pUH84, sous le contrôle du promoteur Lac.

Dans ce cas, le gène E comporte six sites uniques de restriction (localisés sur toute la séquence du gène E) et code pour la gpE qui provoque la lyse cellulaire de E. coli. Dans ce cas, la sélection positive s'effectue lorsque un gène étranger recombinant est venu s'insérer au niveau d'un des sites de restriction.

Cependant, cette insertion d'un gène étranger au niveau d'un site de restriction localisé dans la séquence du gène E, codant pour la gpE, rend plus difficile le séquençage et/ou l'amplification par PCR du gène étranger, car dans ce cas, on séquence, on amplifie et on caractérise également des portions de séquences inutiles appartenant au gène E codant pour la gpE.

Kuhn et al (Gène, vol 42, n°3, 1986, p 253-263) décrivent un vecteur comprenant un gène de grande taille, codant pour une enzyme de restriction qui tue par clivage du genome de la bactérie, ledit gène étant incorporé dans le plasmide pKG2 sous le contrôle du promoteur LacUV5.

Les vecteurs de clonage de l'état de la technique présentent l'inconvénient de devoir être maintenus dans une souche hôte comportant le represseur LacI^{q} sous forme épisomique ou le represseur CI, afin d'inactiver le promoteur d'empêcher l'expression du gène tueur et d'entraîner ainsi la mort de la souche hôte.

Aussi, si l'on désire faire produire par cette souche un grand nombre de copies des vecteurs de clonage, le represseur ne sera pas suffisant pour empêcher soit une pression sélective modifiant l'activité cytotoxique du vecteur, soit une "fuite génétique", c'est-à-dire l'expression de certaines copies du vecteur et la mort de la souche hôte.

Par conséquent, aucun des documents de l'état de la technique ne décrit un vecteur de clonage pouvant incorporer des fragments nucléotidiques de grande taille, qui soit de manipulation aisée et qui puisse être produit de manière industrielle par un microorganisme; c'est-à-dire qu'il puisse être produit en nombre de copies par un microorganisme sans occasionner la mort de celui-ci.

### Buts de l'invention

La présente invention vise à fournir un nouveau vecteur de clonage et/ou de séquençage, ainsi que sa souche hôte, qui soient de construction et de production aisées et peu coûteuses, et qui permettent la sélection directe de clones recombinants, sans présenter les inconvénients de l'état de la technique susmentionné.

Un but particulier de la présente invention est d'obtenir un vecteur qui permet une sélection spécifique et certaine des clones recombinants.

Un autre but de la présente invention vise à obtenir un vecteur qui permet le séquençage, l'amplification et/ou la caractérisation avec la même amorce, de n'importe quel fragment d'ADN étranger (quelque soit sa taille) dans des clones recombinants.

Un but supplémentaire de la présente invention, vise à obtenir un vecteur qui permet également une extraction aisée de ce fragment d'ADN étranger, du clone recombinant.

Un dernier but de la présente invention vise à obtenir une souche hôte dudit vecteur qui permette d'obtenir la production d'un grand nombre de copies dudit vecteur sans occasionner de pression sélective modifiant l'activité cytotoxique dudit vecteur ou la mort de la souche hôte.

### Eléments caractéristiques de l'invention

L'invention concerne un nouveau vecteur de clonage et/ou de séquençage, comprenant, incorporé dans un vecteur à réplication autonome, au moins une séquence nucléotidique promotrice et au moins une séquence nucléotidique codant pour une protéine de fusion active en tant que poison, ladite séquence nucléotidique étant obtenue par la fusion d'une séquence nucléotidique codante comprenant plusieurs sites uniques de clonage et d'une séquence nucléotidique codant pour une protéine poison.

De préférence, le vecteur à réplication autonome est un virus ou un plasmide tel qu'un plasmide pUC, recombinant.

La séquence nucléotidique promotrice peut comprendre n'importe quel promoteur, permettant l'expression de la séquence nucléotidique codant pour une protéine de fusion active en tant que poison.

De préférence, cette séquence nucléotidique promotrice est constituée par le promoteur de l'opéron Lac.

Selon une forme d'exécution préférée de l'invention, les sites uniques de clonage (MCS) de la séquence nucléotidique fusionnée à la séquence nucléotidique codant pour la protéine poison sont absents du reste de la séquence nucléotidique du vecteur selon l'invention.

Avantageusement, la séquence nucléotidique du gène codant pour la protéine poison comprend tout ou une partie de la séquence nucléotidique du gène sauvage codant pour la protéine CcdB.

De préférence, la séquence nucléotidique du gène codant pour la protéine poison est dépourvue du site de clivage de l'enzyme de restriction SmaI.

Un autre aspect de l'invention, concerne une cellule procaryote transformée par le vecteur de clonage selon l'invention.

L'invention concerne également une cellule procaryote hôte du vecteur selon l'invention, comportant un I^{q} chromosomique, un taux de transformation élevé et comportant une mutation conférant la résistance à l'activité poison de la protéine de fusion et/ou comportant un gène codant pour une protéine antipoison de la protéine de fusion.

De préférence, la cellule procaryote hôte du vecteur selon l'invention comporte une mutation dans le gène codant pour la sous unité A ou dans le gène codant pour la sous unité B de la gyrase et conférant la résistance à la protéine de fusion et/ou un gène codant pour la protéine CcdA antipoison de la protéine de fusion.

Préférentiellement, la cellule procaryote est une cellule d'Escherichia coli comportant une mutation responsable de la substitution de l'arginine 462 par une cystéine dans la séquence des acides aminés du polypeptide GyrA de la gyrase, conférant la résistance à la protéine de fusion.

De préférence, cette cellule procaryote hôte comporte également la mutation *LacI*^{*q*}*.*

La présente invention concerne également des fragments du vecteur selon l'invention, en particulier des amorces de séquençages et/ou d'amplification (par exemple par PCR) des fragments nucléotidiques étrangers insérés dans le vecteur selon l'invention.

De préférence, ces amorces sont constituées par des séquences de 10 à 30 nucléotides s'hybridant aux séquences nucléotidiques situées de part et d'autre de la séquence nucléotidique comprenant plusieurs sites uniques de clonage, du vecteur selon l'invention.

Un dernier aspect de l'invention concerne l'utilisation du vecteur selon l'invention pour la sélection et le séquençage de clones recombinants.

### Brève description des figures

- La figure 1 représente de manière schématique un vecteur de clonage selon la présente invention.
- La figure 2 représente la séquence nucléotidique du gène ccdB codant pour la protéine CcdB.
- Les figures 3 et 4 représentent la séquence nucléotidique codant pour la protéine de fusion des vecteurs de clonages pKIL18 et pKIL19 respectivement. Ces séquences sont pourvues d'une séquence nucléotidique contenant de multiples sites uniques de clivage pour différents enzymes de restriction. Ces vecteurs de clonages pKIL18 et pKIL19 ont été obtenus par une recombinaison in vitro entre le gène sauvage ccdB du plasmide F et les plasmides pUC18 et pUC19 respectivement.

### Description d'une forme d'exécution préférée de l'invention

Selon l'invention, le vecteur de clonage et/ou de séquençage 1 comprend incorporé dans un vecteur à réplication autonome 2, au moins une séquence nucléotidique promotrice 3 et au moins une séquence nucléotidique 4 codant pour une protéine de fusion active en tant que poison; ladite séquence nucléotidique 4 étant obtenue par la fusion d'une séquence nucléotidique codante 5 (ou polylinker) comprenant plusieurs (multiples) sites uniques de clonage (MCS) et d'une séquence nucléotidique 6 codant pour une protéine poison.

On entend par vecteur à réplication autonome 2, toute construction nucléotidique telle qu'un virus ou un plasmide (de préférence un plasmide de la série PUC recombinant) susceptible de s'introduire dans un microorganisme, de s'y recombiner et/ou de s'y répliquer.

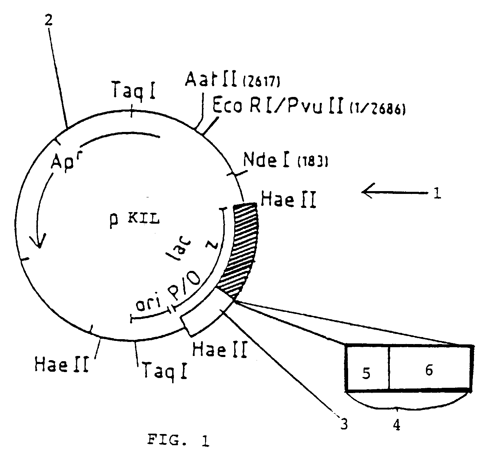

La figure 1 représente de manière schématique un vecteur de clonage selon la présente invention, construit à partir d'un plasmide de la série pUC (pUC18 et pUC19) décrite par Norrander et al (Construction of improved M13 vectors using oligodeoxinucleotide-directed mutagenesis, Gene, 26, p 101-106 (1983)) et par Yanisch-Perron et al (Improved M13 phage cloning vectors and host strains : nucleotide sequences of the M13mp18 and pUC19 vectors, Gene, 33, p 103-119 (1985)).

On entend par séquence nucléotidique codante 5 comprenant plusieurs (multiples) sites uniques de clonage (MCS) une courte séquence codante (ou polylinker) comprenant plusieurs sites de clivage pour des enzymes de restriction.

L'avantage d'un polylinker dans le vecteur selon l'invention est la localisation sur une seule courte séquence de différents sites de clonage qui permet :
- la séquence et l'amplification rapide avec les mêmes amorces, de n'importe quel fragment d'ADN inséré dans ce vecteur.
- l'extraction rapide de fragment cloné, facilitée par la proximité des sites de restriction. En effet, contrairement à l'état de la technique cette proximité évite de séquencer, d'amplifier et de caractériser des fragments inutiles des autres séquences du vecteur selon l'invention.

On entend par séquence nucléotidique 6 codant pour une protéine poison, toute structure nucléotidique (sauvage) codant pour une protéine présentant une activité naturellement poison et spécifique sur une ou plusieurs fonctions vitales d'une cellule hôte.

Une protéine poison est également caractérisée par l'existence d'un antidote ou antipoison, tel que la protéine CcdB et CcdA, la protéine Kid et son antagoniste Kis, la protéine PemK et son antagoniste PemI, la protéine Doc et antagoniste Phd, la protéine HoK et son antogiste Sok et d'autres molécules poisons d'origine plasmidiques ou non.

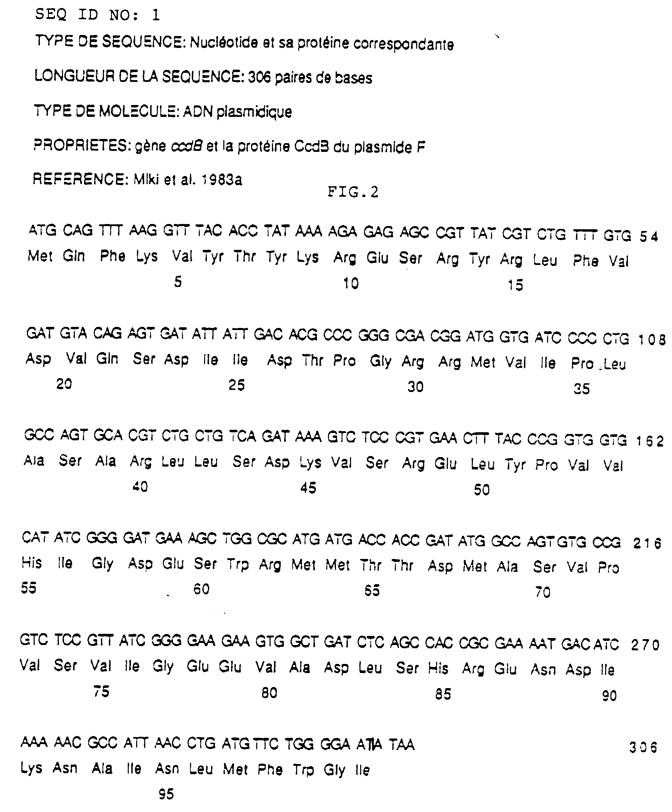

Dans ce cas, la séquence nucléotidique 6 codant pour une protéine poison est constituée par le gène sauvage CcdB codant pour la protéine CcdB (Control of Cell Death) obtenue à partir du locus ccd du plasmide F (figure 2).

Le locus ccd du plasmide F comprend les deux gènes sauvages, ccdA et ccdB, également nommés H et G, ou letA et letD, et qui codent respectivement pour des protéines de 72 et 101 acides aminés (Bex et al, Mini-F encoded proteins; identification of a new 10.5 kilodalton species. EMBO J.2, 1853-1861 (1983); Miki et al, Control of cell division by sex factor F in Escherichia coli. I. The 42.84-43.6 F segment couples cell division of the host bacteria with replication of plasmid DNA, J. Mol. Bio., 174, 605-625,(1984)).

Chez Escherichia coli, la protéine CcdB du plasmide F est une cytotoxine dont l'activité létale est antagonisée par la protéine CcdA (Karoui et al, Ham22, a mini-F mutation which is lethal to host cell and promotes recA-dependent induction of lambdoid prophage. EMBO J.2, 1863-1868 (1983); Ogura and Hiraga Mini-F plasmid gene that couple host cell division to plasmid proliferation, Proc. Natl. Acad. Sci. USA, 80, 4784-4788 (1983); Miki et al, Control of cell division by sex factor F in Escherichia coli. Identification of genes for inhibitor protein and trigger protein on the 42.84-43.6F segment, J. Mol. Biol. 174, 627-646 (1984b)).

Le mécanisme moléculaire par lequel la protéine CcdB exerce son activité létale a été élucidé ; la protéine CcdB est un poison de la DNA-topoisomérase II.

Les DNA topoisomérases de type II sont des enzymes essentiels et ubiquistes qui altèrent la topologie du DNA en introduisant transitoirement une cassure double-brin dans le DNA. Durant l'étape de cassure-religation, la topoisomérase II forme un complexe intermédiaire avec son DNA substrat dans lequelle l'enzyme est attaché de manière covalente à l'extrémité 5' du DNA clivé. Cet intermédiaire transitoire dans lequel la topoisomérase II est liée de manière covalente au DNA a été nommé le "complexe clivable" (Wang, DNA topoisomérases. Annu. Rev. Biochem. 54, 665-97, 1985; Maxwell & Gellert, Mechanistic aspects of DNA topoisomérases. Advan. Protein Chem. 38, 69-107, 1986; Liu, DNA topoisomérase poisons as antitumor drugs, Annu. Rev. Biochem. 58. 351-375, 1989).

Aussi bien chez les eucaryotes que chez les procaryotes, le complexe clivable topoisomérase II-DNA est la cible de puissants agents thérapeutiques, incluant les antibiotiques de la famille des "quinolones", qui agissent sur la gyrase (topoisomérase II bactérienne) et des agents anticancéreux (acridines, epipodophylotoxins), qui agissent sur la topoisomérase II des mammifères. L'efficacité thérapeutique des poisons des topoisomérases est corrélée à leur capacité à stabiliser le complexe clivable.

La DNA-topoisomérase II est une enzyme essentielle chez tous les êtres vivants et très conservée dans l'évolution des espèces. La protéine CcdB présente donc une activité cytotoxique potentielle parmi une grande variété d'espèces procaryotiques.

La petite taille du gène sauvage ccdB permet de l'insérer dans des plasmides sans en augmenter exagérément la taille et permet par conséquent d'y inclure des fragments d'ADN étrangers de grandes tailles. De plus, étant donné sa petite taille, le gène sauvage ccdB du plasmide F contient très peu de sites de restriction, il est donc plus aisé de préserver l'unicité des sites multiples de clonage (MCS) qui lui sont ajoutés.

De manière inattendue, les inventeurs ont constaté que la fusion en phase de la séquence nucléotidique 6 codant pour la protéine CcdB avec la séquence nucléotidique codante (polylinker 5) comprenant plusieurs (multiples) sites uniques de clonage (MCS), donnait une séquence nucléotidique 4 codant pour une protéine de fusion active en tant que poison qui permet par conséquent de produire des vecteurs de sélection directe des plasmides recombinants ("killer selection").

Les plasmides obtenus permettent de cloner les fragments de restriction doublement digérés dans les deux orientations par rapport au promoteur lac. L'insertion d'un fragment de restriction dans un des sites uniques de clonage, interrompt l'information génétique du gène de fusion ce qui mène à la synthèse d'un produit de gène de fusion non fonctionnel. L'inactivation insertionelle du gène de fusion devrait toujours avoir lieu lorsqu'un codon de terminaison est introduit ou lorsqu'un changement de phase lecture est créé.

Les cellules hébergeant un tel vecteur de clonage intact produisent une protéine poison fonctionelle et meurent par l'effet poison de la protéine.

L'insertion d'un fragment d'ADN étranger dans un des sites uniques de clonage du gène de fusion, interfère avec la production du poison.

Les cellules hébergeant un vecteur recombinant seront viables, alors que celles hébergeant un vecteur intact seront non viables. Cette "Killer Selection" par simple culture sur milieu solide permet d'éliminer les cellules hébergeant un vecteur non recombinant (clones non viables) et de sélectionner les clones recombinants (clones viables).

### Exemple I : Construction du Plasmide pKIL19

Le gène ccdB a été amplifié par PCR en utilisant comme ADN matrice le plasmide pULB2208 (Bernard and Couturier, The 41 carboxy-terminal residues of the miniF plasmid CcdA protein are sufficient to antagonize the killer activity of the CcdB protein, Mol. Gen. Genet. 226, 297-304 (1991)) ainsi que des oligonucléotides synthétiques.

Les séquences des oligonucléotides synthétiques ont été choisies de manière à créer un site de restriction EcoRI de part et d'autre du gène sauvage ccdB afin de pouvoir recloner ce gène en phase avec les codons des sites multiples de clonage MCS19 et d'éliminer le codon d'initiation du gène ccdB natif. L'ADN issu de la réaction PCR a été digéré par l'enzyme EcoRI et cloné dans le site EcoRI du plasmide pUC19. Le plasmide résultant, ayant intégré le fragment EcoRI dans l'orientation permettant la lecture à partir du promoteur *Lac* du gène ccdB doté des codons additionels correspondants aux sites multiples de clonage MCS19, a été appelé pKIL2. Le plasmide pKIL2 est létal pour une bactérie sauvage (sensible CcdB^{S}).

Le pKIL2 possède encore deux sites SmaI, l'un au niveau des multiples sites de clonage, l'autre dans la région centrale du gène ccdB. Ce dernier a été éliminé par mutagénèse site spécifique. Le plasmide résultant pKIL3, pourvu d'un unique site SmaI est encore doté de deux sites EcoRI. Le site EcoRI en aval du gène ccdB a été éliminé par remplissage de ses extrémités cohésives.

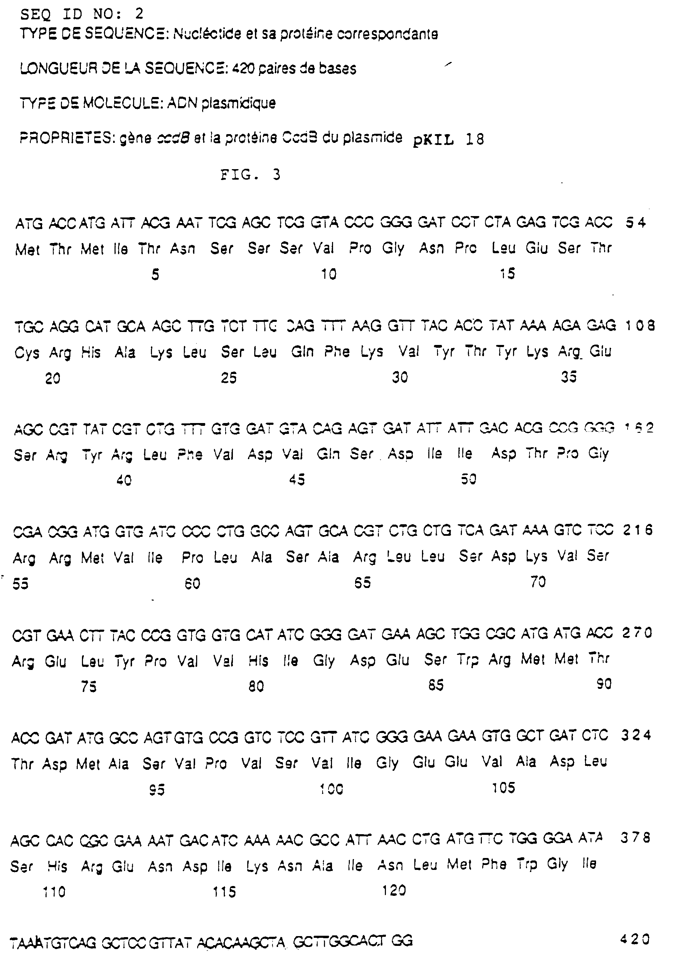

Le plasmide résultant, pKIL19 (figure 3) possède donc un site de restriction EcoRI unique au niveau de la séquence 5 comprenant les multiples sites de clonage.

### Exemple II : construction du plasmide pKIL18

Le gène ccdB a été amplifié par PCR en utilisant comme DNA matrice le plasmide pKIL19 ainsi que des oligonucléotides synthétiques. Les séquences des oligonucléotides synthétiques ont été choisies de manière à créer un site HindIII de part et d'autre du gène ccdB afin de pouvoir recloner ce gène en phase avec les codons des sites multiples de clonage MCS18. L'ADN issu de la réaction de PCR a été digéré par l'enzyme HindIII et cloné dans le site HindIII du plasmide pUC18. Le plasmide résultant, ayant intégré le fragment HindIII dans l'orientation permettant la lecture à partir du promoteur *Lac* du gène ccdB dotés des codons additionels correspondants aux sites multiples de clonage MCS18, a été appelé pKIL4. Le plasmide pKIL4 est létal pour une bactérie sensible CcdB^{S}.

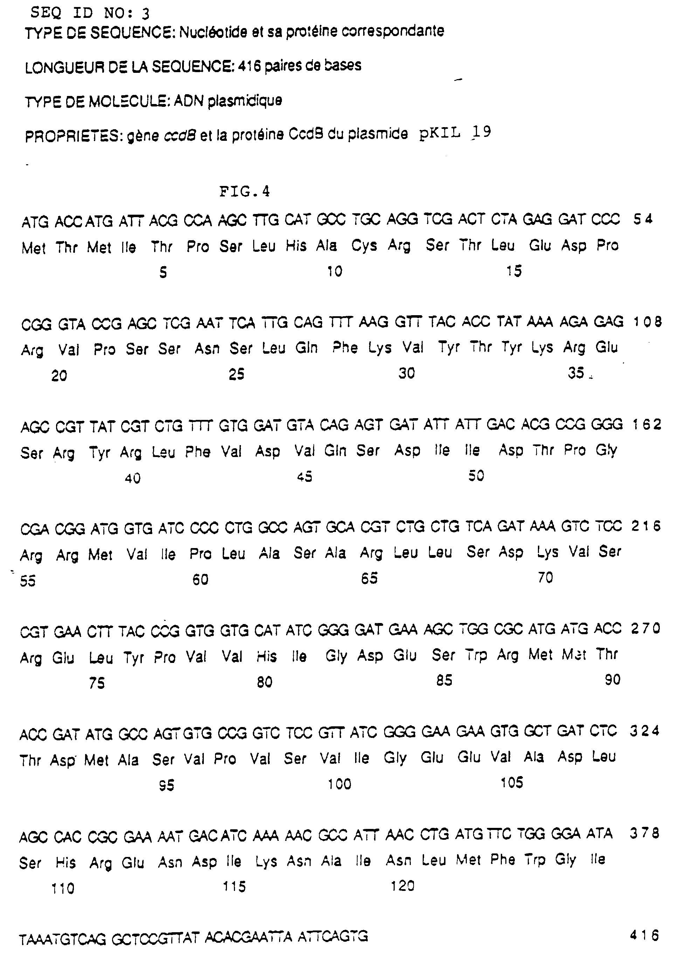

Le site HindIII en aval du gène ccdB a été éliminé par remplissage de ses extrémités cohésives. Le plasmide résultant pKIL18 (figure 4), possède une site unique de restriction HindIII ainsi qu'un site SmaI unique (car construit à partir du pKIL19).

### Exemple III : Construction des souches CcdB^{r} et CcdB^{S}

Pour pouvoir maintenir les plasmides pKIL18 et pKIL19 au sein d'une bactérie, celle-ci doit être résistante à l'effet létal de la protéine de fusion active en tant que poison. De manière inattendue, la mutation chromosomique *gyr*A462 confère aux souches une résistance totale à l'effet poison de la protéine fusion.

D'autre part, les plasmides pKIL18 et pKIL19 dérivant directement des plasmides pUC18 et pUC19 et exprimant les gènes ccdB à partir du promoteur *Lac,* il est préférable de maintenir ces plasmides dans une souche *LacI*^{q}*.* En effet, dans notre cas, une surexpression continuelle de ces gènes ne joue pas une pression de sélection en faveur de certaines mutations, mais la souche *LacI*^{q} permet de réduire l'expression à partir du promoteur Lac et économise la machinerie bactérienne et ce qui permet de garantir un temps de génération rapide (production élevée du vecteur par la souche).

La souche D1210 (Sadler et al Gène 8, p 279-300 (1980)) issue de la souche HB101 *LacI*^{q}, *LacY*⁺ (Maniatis et al (Molecular Cloning Laboratories Man. Cold Spring Harbor Laboratory N.Y.), caractérisée par un I^{q} chromosomique et un taux de transformation élevé, a été transformée par le plasmide pC0S2.1. Ce plasmide, qui confère la résistance à la kanamycine, porte le gène recA d'Erwinia chrysanthemi 3665, et permet la recombinaison chez E. coli. Un lysat de phage Pl a été préparé sur une souche CcdB^{R} *gyr*A462, zei-298:: Tn10 et utilisé pour infecter la souche D1210/pCOS2.1. Les transductants résistants à la tétracycline ont été sélectionnés et testés pour leur résistance ou sensibilité à la protéine CcdB. L'un des transductants CcdB^{R} a ensuite été curé du plasmide pCOS2.1 et dénommé KIB22.

La souche KIB22 constitue une souche hôte idéale pour les plasmides pKIL18 et pKIL19 alors que la souche D1210 constitue l'hôte idéal pour la sélection des plasmides recombinants.

En effet, la souche KIB22 possède de manière avantageuse un taux d'extraction de l'ADN élevé (comparable au rendement des plasmides pUC) et de manière inattendue une résistance à la protéine de fusion codée par le pKIL18 et pKIL19.

Par conséquent, il est possible de produire de manière industrielle par ce microorganisme, le vecteur de clonage selon l'invention en de nombreuses copies sans occasionner la mort dudit microorganisme.

La sélection se fait par simple étalement des bactéries sur milieu contenant de l'IPTG (Isopropyl-Beta-D-thiogalacto-pyranoside), ainsi que de l'ampicilline.

La souche KIB22 a été déposée auprès du Laboratorium voor Microbiologie-Bacteriënverzameling (LMG) des Belgian Coordinated Collections of Microorganisms (BCCM) sous le n°LMG P-12601.

Le vecteur de clonage pKIL19 a été déposé auprès du Laboratorium voor Moleculaire Biologie-Plasmiden Collectie (LMBP) des Belgian Coordinated Collections of Microorganisms (BCCM) sous le n° LMBP 2781.

Ces dépôts ont été faits selon les dispositions du Traité de Budapest sur la Reconnaissance Internationale du Dépôt de Microorganismes.

## Revendications

1. Vecteur de clonage et/ou de séquençage caractérisé en ce qu'il comprend, incorporé dans un vecteur à réplication autonome (2), au moins une séquence nucléotidique promotrice (3) et au moins une séquence nucléotidique (4) codant pour une protéine de fusion active en tant que poison, ladite séquence nucléotidique (4) étant obtenue par la fusion d'une séquence nucléotidique codante (5) comprenant plusieurs sites uniques de clonage et d'une séquence nucléotidique (6) codant pour une protéine poison.

2. Vecteur selon la revendication 1 caractérisé en ce que le vecteur à réplication autonome (2) est un virus recombinant.

3. Vecteur selon la revendication 1 caractérisé en ce que le vecteur à réplication autonome (2) est un plasmide recombinant.

4. Vecteur selon la revendication 3 caractérisé en ce que le vecteur à réplication autonome (2) est un plasmide pUC recombinant.

5. Vecteur selon l'une quelconque des revendications précédentes caractérisé en ce que la séquence nucléotidique promotrice (3) est constituée par le promoteur de l'opéron lac.

6. Vecteur selon l'une quelconque des revendications précédentes caractérisé en ce que les sites uniques de clonage de la séquence nucléotidique (5) fusionnée à la séquence nucléotidique (6) codant pour la protéine poison, sont absents du reste de la séquence nucléotidique du vecteur de clonage.

7. Vecteur selon l'une quelconque des revendications précédentes caractérisé en ce que la séquence nucléotidique (6) codant pour une protéine poison comprend tout ou une partie de la séquence nucléotidique du gène sauvage codant pour la protéine CcdB.

8. Vecteur selon la revendication 7 caractérisé en ce que la séquence nucléotidique (6) codant pour la protéine poison est dépourvue du site de clivage de l'enzyme de restriction SmaI.

9. Vecteur selon l'une quelconque des revendications précédentes caractérisé en ce qu'il correspond au dépôt n° LMBP2781.

10. Cellule procaryote transformée par le vecteur de clonage selon l'une quelconque des revendications précédentes.

11. Cellule procaryote comprenant le vecteur de clonage selon l'une quelconque des revendications précédentes 1 à 9, caractérisée en ce qu'elle comporte de préférence un I^{q} chromosomique , un taux de transformation élevé et comporte une mutation conférant la résistance à l'activité poison de la protéine de fusion et/ou comporte un gène codant pour une protéine antipoison de la protéine de fusion.

12. Cellule procaryote selon la revendication 11 caractérisé en ce qu'elle comporte une mutation dans le gène codant pour la sous unité A ou dans le gène codant pour la sous unité B de la gyrase et conférant la résistance à la protéine de fusion et/ou comporte un gène codant pour la protéine CcdA antipoison de la protéine de fusion.

13. Cellule selon la revendication 11 ou 12 caractérisée en ce qu'elle est une cellule d'Escherichia coli comportant une mutation responsable de la substitution de l'arginine 462 par une cystéine dans la séquence des acides aminés du polypeptide GyrA de la gyrase conférant la résistance à la protéine de fusion.

14. Cellule selon l'une quelconque des revendications 11 à 13 caractérisé en ce qu'elle comporte la mutation *LacI*^{q}.

15. Cellule selon l'une quelconque des revendications précédentes 11 à 14 caractérisée en ce qu'elle est déposée sous le n° LMGP-12601.

16. Utilisation du vecteur de clonage selon l'une quelconque des revendications 1 à 9 pour la sélection de clones recombinants.

## Patentansprüche

1. Klonierungs- und/oder Sequenzierungsvektor, dadurch gekennzeichnet, daß er, eingebaut in einen Vektor (2) mit autonomer Replikation, mindestens eine Promotor-Nukleotidsequenz (3) und mindestens eine Nukleotidsequenz (4), die für ein als Poison wirkendes Fusionsprotein kodiert, aufweist, wobei die Nukleotidsequenz (4) durch die Fusion einer kodierenden Nukleotidsequenz (5), die mehrere einzelne Klonierungsstellen aufweist, und mit einer für ein Poisonprotein kodierenden Nukleotidsequenz (6) erhalten wird.

2. Vektor gemäß Anspruch 1, dadurch gekennzeichnet, daß der Vektor (2) mit autonomer Replikation ein rekombinanter Virus ist.

3. Vektor gemäß Anspruch 1, dadurch gekennzeichnet, daß der Vektor (2) mit autonomer Replikation ein rekombinantes Plasmid ist.

4. Vektor gemäß Anspruch 3, dadurch gekennzeichnet, daß der Vektor (2) mit autonomer Replikation ein rekombinantes pUC-Plasmid ist.

5. Vektor gemäß irgendeinem der vorhergehenden Ansprüche, dadurch gekennzeichnet, daß die Promotor-Nukleotidsequenz (3) aus dem Promotor des Operons lac besteht.

6. Vektor gemäß irgendeinem der vorhergehenden Ansprüche, dadurch gekennzeichnet, daß die einzelnen Klonierungsstellen der Nukleotidsequenz (5), die mit der für das Poisonprotein kodierenden Nukleotidsequenz (6) fusioniert ist, bei dem Rest der Nukleotidsequenz des Klonierungsvektors fehlen.

7. Vektor gemäß irgendeinem der vorhergehenden Ansprüche, dadurch gekennzeichnet, daß die für ein Poisonprotein kodierende Nukleotidsequenz (6) die Nukleotidsequenz des für das Protein CcdB kodierenden, wilden Gens ganz oder teilweise aufweist.

8. Vektor gemäß Anspruch 7, dadurch gekennzeichnet, daß die für das Poisonprotein kodierende Nukleotidsequenz (6) die Spaltungsstelle für das Restriktionsenzym SmaI nicht aufweist.

9. Vektor gemäß irgendeinem der vorhergehenden Ansprüche, dadurch gekennzeichnet, daß er der Anmeldung Nr. LMBP2781 entspricht.

10. Prokaryontenzelle, transformiert durch den Klonierungsvektor gemäß irgendeinem der vorhergehenden Ansprüche.

11. Prokaryontenzelle, die den Klonierungsvektor gemäß irgendeinem der vorhergehenden Ansprüche 1 bis 9 aufweist, dadurch gekennzeichnet, daß sie vorzugsweise einen Chromosomen-I^{q} und einen hohen Transformationsgrad umfaßt, und eine Mutation umfaßt, die die Widerstandsfähigkeit gegen die Poisonwirksamkeit des Fusionsproteins verleiht, und/oder ein Gen umfaßt, das für ein Antipoisonprotein des Fusionsproteins kodiert.

12. Prokaryontenzelle gemäß Anspruch 11, dadurch gekennzeichnet, daß sie eine Mutation bei dem für die Untereinheit A kodierenden Gen, oder bei dem für die Untereinheit B kodierenden Gen der Gyrase umfaßt, das die Widerstandsfähigkeit gegen das Fusionsprotein verleiht, und/oder ein für das Antipoisonprotein CcdA des Fusionsproteins kodierendes Gen umfaßt.

13. Zelle gemäß Anspruch 11 oder 12, dadurch gekennzeichnet, daß sie eine Zelle von Escherichia coli ist, die eine Mutation umfaßt, die für die Substitution des Arginins 462 durch ein Zystein bei der Sequenz der Aminosäuren des Polypeptids GyrA der Gyrase verantwortlich ist, und die Widerstandsfähigkeit gegen das Fusionsprotein verleiht.

14. Zelle gemäß irgendeinem der Ansprüche 11 bis 13, dadurch gekennzeichnet, daß sie die Mutation LacI^{q} umfaßt.

15. Zelle gemäß irgendeinem der vorhergehenden Ansprüche 11 bis 14, dadurch gekennzeichnet, daß sie unter der Nr. LMGP-12601 angemeldet ist.

16. Verwendung des Klonierungsvektors gemäß irgendeinem der Ansprüche 1 bis 9 zur Selektion von rekombinanten Klonen.

## Claims

1. Cloning and/or sequencing vector which is characterized in that it includes, incorporated into an autonomously replicating vector (2), at least one promoter nucleotide sequence (3) and at least one nucleotide sequence (4) which encodes a fusion protein which is active as a poison, the said nucleotide sequence (4) being obtained by fusing a coding nucleotide sequence (5), which comprises several unique cloning sites, and a nucleotide sequence (6) which encodes a protein poison.

2. Vector according to Claim 1, characterized in that the autonomously replicating vector (2) is a recombinant virus.

3. Vector according to Claim 1, characterized in that the autonomously replicating vector (2) is a recombinant plasmid.

4. Vector according to Claim 3, characterized in that the autonomously replicating vector (2) is a recombinant pUC plasmid.

5. Vector according to any one of the preceding claims, characterized in that the promoter nucleotide sequence (3) consists of the *lac* operon promoter.

6. Vector according to any one of the preceding claims, characterized in that the unique cloning sites of the nucleotide sequence (5), which is fused to the nucleotide sequence (6) which encodes the protein poison, are absent on the remainder of the nucleotide sequence of the cloning vector.

7. Vector according to any one of the preceding claims, characterized in that the nucleotide sequence (6) which encodes a protein poison comprises all or part of the nucleotide sequence of the wild-type gene which encodes the protein CcdB.

8. Vector according to Claim 7, characterized in that the nucleotide sequence (6) which encodes the protein poison lacks the cleavage site for the restriction enzyme SmaI.

9. Vector according to any one of the preceding claims, characterized in that it corresponds to deposition No. LMBP2781.

10. Procaryotic cell which is transformed with the cloning vector according to any one of the preceding claims.

11. Procaryotic host cell comprising the cloning vector according to any one of the preceding Claims 1 to 9, characterized in that it preferably possesses a chromosomal I^{q} and an increased transformation efficiency and possesses a mutation which confers resistance to the poisonous activity of the fusion protein and/or possesses a gene which encodes a protein which is an antipoison to the fusion protein.

12. Procaryotic host cell according to Claim 11, characterized in that it possesses a mutation in the gene encoding subunit A or in the gene encoding subunit B of the gyrase, conferring resistance to the fusion protein, and/or possesses a gene which encodes the protein CcdA which is the antipoison to the fusion protein.

13. Cell according to Claim 11 or 12, characterized in that it is a Escherichia coli possessing a mutation which is responsible for replacing arginine 462 with a cysteine in the amino acid sequence of the GyrA polypeptide of the gyrase, thereby conferring resistance to the fusion protein.

14. Cell according to any one of Claims 11 to 13, characterized in that it possesses the *Lacl*^{*q*} mutation.

15. Cell according to any one of the preceding Claims 11 to 14, characterized in that it is deposited under No. LMGP-12601.

16. Use of the cloning vector according to any one of Claims 1 to 9 for selecting recombinant clones.
